# EUROPÄISCHE PATENTSCHRIFT

(11) **EP 0 543 272 B1**
(45) Veröffentlichungstag und Bekanntmachung des Hinweises auf die Patenterteilung: **07.02.1996**
(21) Anmeldenummer: 92119294.4
(22) Anmeldetag: 11.11.1992
(51) Int. Cl.: A61B 17/02, A61B 17/00, A61B 19/00

(54) **Adhäsionsprophylaxe**
Adhesion prophylaxis
Prophylaxie d'adhésion

(30) Priorität: 19.11.1991 DE 4138100
(43) Veröffentlichungstag der Anmeldung: 26.05.1993
(73) Patentinhaber: WISAP GESELLSCHAFT FÜR WISSENSCHAFTLICHEN APPARATEBAU MBH, D-82054 Sauerlach (DE)
(72) Erfinder: Semm, Kurt, o.Prof. Dr.med. Dr.med.vet.h.c., W-2300 Kiel (DE)
(74) Vertreter: Heim, Hans-Karl, Dipl.-Ing.

(56) Entgegenhaltungen:
- EP-A- 0 213 563
- EP-A- 0 252 607
- EP-A- 0 411 767
- US-A- 4 291 687
- US-A- 4 549 529
- US-A- 5 033 481

## Beschreibung

Die Erfindung betrifft eine Vorrichtung zur mechanischen Adhäsionsprophylaxe, insbesondere für die endoskopische Abdominalchirurgie, gemäß Oberbegriff des Anspruchs 1.

Während die Operationstechniken im Bauchraum in Bezug auf die Organbehandlung einen extrem hohen Standard erreicht haben, existiert noch keine sichere Methode zur Vermeidung postoperativer Verwachsungen im Darm- und Netzbereich. Bislang erreicht keine Methode eine Erfolgsrate, die bei einer Re-Laparotomie, d.h. einer wiederholten Leibeseröffnung, eine Adhäsiolyse, d.h. eine Lösung von Darm- und Netzverwachsungen, erfolgreich durchführen läßt. Man beschränkt sich daher in der Allgemeinchirurgie in der Regel darauf, sogenannte Adhäsiolysen nur bei extremen Beschwerden, z.B. Subileus oder erneuten akutem Darmverschluß, durchzuführen. Die Rezidivrate liegt im Bereich von 80%. Dies bedeutet, daß durchaus die Gefahr besteht, daß die Anzahl der Verwachsungen nach einem derartigen Eingriff noch zunimmt.

In der EP 213 563 A3 ist als Material für eine Adhäsionsprophylaxe ein Gewebe aus einem oxidierten und regenerierten Zellulosematerial oder aus einem weiteren bioresorbierbaren Material beschrieben, welches eine definierte Porosität aufweist.

Aus dem US-PS 5,033,481 ist ein Gewebeexpander bekannt, der für Blutgefäße und ähnliche Lineargewebe verwendet wird. Die für einen operativen Eingriff zu verlängernden Gefäße werden mit Hilfe eines Fixierelementes gehalten, welches auf dem aufblasbaren, als ein Rundkissen ausgebildeten Expander befestigt ist und das Gefäß umhüllt.

Es ist daher **Aufgabe** der Erfindung, eine mechanische Adhäsionsprophylaxe zur Vermeidung von postoperativen Verwachsungen zu schaffen, die ein Verkleben und Aneinanderwachsen peritonealisierter Gewebeflächen unterbindet und insbesondere in der endoskopischen Abdominalchirurgie verwendet und relativ einfach appliziert werden kann.

Diese Aufgabe wird durch eine Vorrichtung zur mechanischen Adhäsionsprophylaxe gemäß Anspruch 1 gelöst. Vorteilhafte Weiterbildungen der Erfindung sind Gegenstand der Unteransprüche.

Erfindungsgemäß wird die Aufgabe durch eine Vorrichtung mit einer aufblasbaren kissenartigen Hülle, einem an der Hülle angeordneten Einblasbereich zum Aufblasen der Hülle und mindestens einem Fixierungsbereich zum Festlegen der Hülle am Körpergewebe gelöst, welche als Hülle einen flächig gestreckten Flachballon aufweist, der insbesondere für die endoskopische Abdominalchirurgie geeignet ist.

Die erfindungsgemäße Vorrichtung zur mechanischen Adhäsionsprophylaxe wird nach dem operativen Eingriff, insbesondere nach einer Adhäsiolyse, d.h. einer mechanischen stumpfen oder scharfen Durchtrennung von verklebten Bauchfellteilen mittels Schere und Messer temporär eine Plastikfolie zwischen aneinander anliegenden Gewebepartien eingelegt. Diese Hülle oder Plastikfolie, die als flächig gestreckter Flachballon ausgebildet ist, verhindert Verklebungen der peritonealisierten Darm-, Netz- und Bauchfellflächen, die durch das Ausschwitzen von Fibrin, Hystoziten und Fibrozyten nach einem derartigen Eingriff sofort wieder auftreten. Nach einer gewissen Zeit von einigen Stunden bis Tagen, vorzugsweise 48 Stunden, wird der Flachballon entfernt. Die Folie des Flachballon soll inert sein, d.h. keine toxischen Eigenschaften besitzen.

Die Folien werden mittels eines speziellen Applikationssets z.B. mittels eines 10 mm Folienapplikators gemäß der deutschen Patentanmeldung P 41 32 855.8 in ein Pneumoperitoneum appliziert. Das Ausbreiten der Folie im Bauchraum nach dem Abwickeln des Einführungsstabs ist jedoch technisch nicht einfach und daher zeitraubend. Das Einlegen der Folie nach der Adhäsiolyse wird dadurch erleichtert, daß die Folie als aufblasbarer kissenartiger Ballon ausgebildet ist. Dieser nimmt dann in der meist durch Kohlensäuregas aufgeblasenen Bauchhöhle die ihm vorgegebene Form an, wie dies z.B. bei einer Luftmatratze der Fall ist.

Ähnlich einer Luftmatratze ist die Intraabdominalfolie abgekammert und so der Bauchhöhlenform insgesamt oder in einzelnen Bereichen derart angepaßt, daß sie sich entsprechend ihrer vorgegebenen Form in der Bauchhöhle ausbreitet, ohne daß hierzu viele Handgriffe notwendig wären. In den meisten Fällen erspart man sich hierdurch das Fixieren des Ballons. Dies kann somit auf Sonderfälle beschränkt werden.

Durch während der Liegezeit intermittierendes Ablassen des Kohlensäuregases aus dem Ballon und durch ein anschließendes Wiederfüllen wird der Effekt der mechanischen Bauchfelltrennung durch den Ballon erhöht. Nach gegebener Zeit läßt sich die Folie bzw. der Ballon an der vorgesehenen Stelle durch einen Trokar durch die Bauchdecke entfernen.

Diese Adhäsionsprophylaxe ist zwar primär für den Abdominalraum entwickelt. Eine Verwendung ist jedoch auch für andere Körperhöhlen möglich. Die Außenkontur des Ballons bzw. der Plastiktasche ist jedoch der entsprechenden Körperhöhle in Größe und Form anzupassen. Eine besonders ausgebildete, insbesondere aufwickelbare bzw. faltbare, Form für das Einblaselement für das Gas garantiert ein Entfernen der Folie ohne einen besonderen Eingriff.

Durch die Verwendung der mechanischen Adhäsionsprophylaxe wird eine starke Verringerung der Rezidivrate verglichen mit der klassischen Adhäsiolyse per laparotomiam erreicht.

Während einfache, einlagige Adhäsionsfolien nach dem Abschätzen der ungefähr erforderlichen Foliengröße vor dem Applizieren mit der Schere zurechtgeschnitten werden, werden aufblasbare Flachballons in verschiedenen Größen hergestellt, die auf die gleiche Art und Weise wie die Folie in das Abdomen eingeführt werden.

Der Ballon kann ein Einblaselement aufweisen und z.B. durch eine Veressnadel aufgepumpt werden. Es kann jedoch auch durch eine zweckmäßig gelegte Trokarhülse ein Aufpumpinstrument, z.B. ein Schlauch, eingeführt werden, das an seinem distalen Ende einen dem Einblaselement komplementären Adapteranschluß aufweist.

Es empfiehlt sich eventuell, oberhalb und unterhalb des applizierten Flachballons je einen Robinson-Katheter oder ähnliche Drainagen einzulegen, um einerseits den Sekretabfluß zu fördern und andererseits bei einer eventuellen Nahtinsuffizienz, z.B. von Darmnähten, sofort die nötige Information über den Sekretbeutel zu erhalten.

Nach 48 Stunden kann beispielsweise die Folie bzw. der aufgeblasene Flachballon mittels einer Second-look-Pelviskopie gegebenenfalls von ihren Fixierstellen gelöst und durch eine Trokarhülse mit 10 mm Durchmesser durch die Bauchdecke in toto herausgezogen werden.

Da durch die Verwendung eines Flachballons die Fixierung der Adhäsionsprophylaxe eventuell unterbleiben kann, erspart man sich die Arbeitsgänge des Fixierens bei der Applizierung bzw. des Lösens beim Entfernen des Flachballons aus der Körperhöhle.

Die Erfindung wird nachfolgend beispielsweise anhand der schematischen Zeichnung beschrieben. In dieser zeigen:
- Fig. 1: eine Aufsicht auf einen Antiadhäsionsbeutel für große Becken und
- Fig. 2: einen Antiadhäsionsbeutel für kleine Becken.

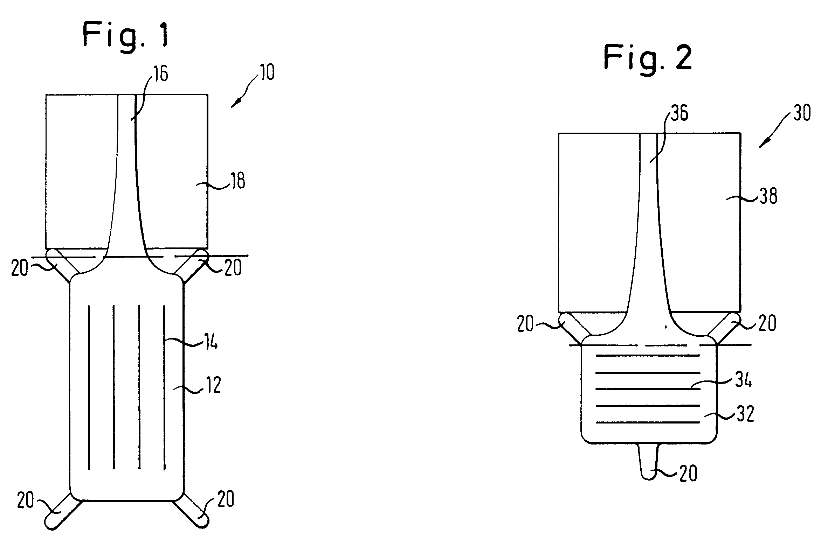

Der in Fig. 1 dargestellte Antiadhäsionsbeutel bzw. die Antiadhäsionsblase 10 besteht aus einem länglichen Luftkissen 12 mit im wesentlichen rechteckiger Grundform, das parallel zur Längsachse verlaufende Versteifungslamellen 14 in der Art einer Luftmatratze aufweist. Eine Stirnseite mündet in einen Einblaskanal 16, der mit einer vierlagigen Folie 18 verschweißt ist, um eine größere Steifigkeit zu erhalten. An seinen vier Ecken weist das Luftkissen 12 Fixierlamellen 20 auf, mit denen der Antiadhäsionsbeutel 10 im Beckenbereich bzw. in der Bauchhöhle fixiert werden kann. Der Antiadhäsionsbeutel bzw. die Antiadhäsionsblase besteht aus Kunststoffolien, die an ihren Rändern miteinander verschweißt sind. Das Material der Kunststoffolie ist nicht toxisch, gewebefreundlich, reißfest, hochflexibel und lagert nur wenig Wasser ein.

Fig. 2 zeigt einen Antiadhäsionsbeutel 30 für kleine Becken. Er besteht aus einem im Vergleich zum Luftkissen 12 aus Fig. 1 kleineren Luftkissen 32, das im wesentlichen die Form einer Tasche hat. Das Luftkissen 32 ist mit quer zur Längsachse des Antiadhäsionsbeutels 30 angeordneten Versteifungslamellen 34 in der Art einer Luftmatratze versehen und weist einen Einblasschlauch 36 auf, der trichterförmig in das Luftkissen 32 übergeht.

Der Einblasschlauch 36 z.B. ist mit einer vierlagigen Folie 38 verschweißt, um die Stabilität des Einblasschlauches bzw. -rohres 36 zu erhöhen.

Die in Fig. 1 und 2 dargestellte Versteifung der Einblasschläuche bzw. Rohre 16,36 durch die Folien 18,38 wird mit dem Luftkissen 12,32 so getroffen, daß nach unten hin ein sich trichterartig öffnender Einströmungsbereich für das einzubringende Medium gebildet wird. Mittig, zentral ist daher eine Art Rohrbildung vorhanden. Die seitlichen Flächenbereiche dienen zur Stabilisierung.

Die Antiadhäsionsbeutel bzw. Antiadhäsionsballons können z. B. über einen separat durch die Trokarhülse einzubringenden Schlauch, der mit dem Mündungsbereich des Einblasschlauchs 16,36 in Verbindung gebracht wird, aufgeblasen werden. Alternativ könnte das den Einblaskanal 16 bzw. 36 aufweisende Ballonende auch durch eine zweckmäßig gelegte Trokarhülse herausgeführt werden.

Die Einführung eines Antiadhäsionsbeutels in das Pneumoperitoneum ist ebenso einfach möglich wie die Applizierung einer einzelnen Folie. So sind die Antiadhäsionsbeutel 10,30 aufrollbar, um auf einen längs geschlitzten Folienapplikator aufgerollt und durch die Trokarhülse eingebracht zu werden. Die Faltlinie zum Aufrollen des Applikators ist gestrichelt angedeutetet und liegt in etwa am Ende des Mündungsbereichs des Einblasschlauchs 16,36.

Anstelle der Vierlagigkeit der Folie mit entsprechender Verschweißung sowohl zum Einfüllschlauch bzw. -rohr wie auch zum eigentlichen beutelartigen Luftkissen könnte auch eine stärkere stabilisierende Folie im Versteifungsbereich des Einblasschlauches 16,36 benutzt werden.

Die Längen von Beutel und Zuführungsbereichen (Einblasschlauch) können selbstverständlich je nach anatomischen und operativen Gegebenheiten unterschiedlich gehalten werden. In der Regel werden Antiadhäsionsbeutel in unterschiedlichen Formen für unterschiedliche Einsatzzwecke vorrätig gehalten.

## Patentansprüche

1. Vorrichtung zur mechanischen Adhäsionsprophylaxe, insbesondere für die endoskopische Abdominalchirurgie, mit einer aufblasbaren kissenartigen Hülle (12), einem an der Hülle (12) angeordneten Einblasbereich (16) zum Aufblasen der Hülle (12) und mindestens einem Fixierungsbereich (20) zum Festlegen der Hülle (12) am Körpergewebe,
dadurch **gekennzeichnet**,
daß die Hülle (12) als flächig gestreckter Flachballon ausgebildet ist.

2. Vorrichtung nach Ansprch 1,
dadurch **gekennzeichnet**,
daß der Flachballon (12) in Eckbereichen die Fixierungsbereiche (20) aufweist.

3. Vorrichtung nach Anspruch 1 oder 2,
dadurch **gekennzeichnet**,
daß der Flachballon (12) aus mindestens zwei wenigstens im Randbereich miteinander verbundenen, insbesondere verschweißten, Folien besteht.

4. Vorrichtung nach einem der Ansprüche 1 bis 3,
dadurch **gekennzeichnet**,
daß der Flachballon (12) durch Versteifungslamellen (14) in der Art einer Luftmatratze abgekammert ist.

5. Vorrichtung nach einem der Ansprüche 1 bis 4,
dadurch **gekennzeichnet**,
daß die Außenkontur des Flachballons (12) im wesentlichen der Kontur des Körperhohlraums entspricht, in dem die Vorrichtung zur Adhäsionsprophylaxe (10) zu applizieren ist.

6. Vorrichtung nach einem der vorhergehenden Ansprüche,
dadurch **gekennzeichnet**,
daß am Flachballon (12) Lamellen oder Folienstreifen als Fixierungsbereiche (20) ausgebildet sind.

7. Vorrichtung nach einem der vorhergehenden Ansprüche,
dadurch **gekennzeichnet**,
daß die Hülle (12) länglich ausgebildet ist und parallel zu ihrer Längsachse angeordnete Versteifungslamellen (14) aufweist.

8. Vorrichtung nach einem der vorhergehenden Ansprüche,
dadurch **gekennzeichnet**,
daß die Hülle (12) aus einer reißfesten, gewebefreundlichen und nur wenig Wasser einlagernden Kunststoffolie besteht.

9. Vorrichtung nach einem der vorhergehenden Ansprüche,
dadurch **gekennzeichnet**,
daß an der Hülle (12) als Einblasbereich (16, 36) ein Einblasschlauch (16, 36) ausgebildet ist.

10. Vorrichtung nach Anspruch 9,
dadurch **gekennzeichnet**,
daß der Einblasschlauch (16, 36) trichterförmig in die Hülle (12, 32) übergeht.

11. Vorrichtung nach einem der vorhergehenden Ansprüche,
dadurch **gekennzeichnet**,
daß die Fixierungsbereiche (20) Clips zur Festlegung am Körpergewebe aufweisen.

## Claims

1. Apparatus for a mechanical adhesion prophylaxis in particular for endoscopic abdominal surgery with an inflatable, cushion-like envelope (12), an inflating area (16) for inflating the envelope (12) and located on the latter and at least one fixing area (20) for fixing the sleeve (12) to a body tissue,
**characterized** in that
the envelope (12) is constructed as a flat stretched flat balloon.

2. Apparatus according to claim 1,
**characterized** in that
the flat balloon (12) has the fixing areas (20) in corner areas.

3. Apparatus according to claim 1 or 2,
**characterized** in that
the flat balloon (12) comprises at least two sheets interconnected, in particular welded together, at least in the marginal area.

4. Apparatus according to anyone of the claims 1 to 3,
**characterized** in that
the flat ballon (12) is compartmentalized in the manner of an air mattress by stiffening plates (14).

5. Apparatus according to anyone of the claims 1 to 4,
**characterized** in that
the outer contour of the flat balloon (12) substantially corresponds to the contour of the body cavity to which the adhesion prophylaxis (10) is to be applied.

6. Apparatus according to anyone of the preceding claims,
**characterized** in that
on the flat balloon (12) plates or sheet strips are constructed as fixing areas (20).

7. Apparatus according to anyone of the preceding claims,
**characterized** in that
the envelope (12) is elongated and has stiffening plates (14) parallel to its longitudinal axis.

8. Apparatus according to anyone of the preceding claims,
**characterized** in that
the envelope (12) is made from a tear-resistant, tissue-friendly plastic sheet, which only stores little water.

9. Apparatus according to anyone of the preceding claims,
**characterized** in that
an inflating hose (16,36) is constructed at the envelope (12) as an inflating area (16,36).

10. Apparatus according to claim 9,
**characterized** in that
the inflating hose (16, 36) passes in funnel-shaped manner into the sleeve (12, 32).

11. Apparatus according to anyone of the preceding claims,
**characterized** in that
the fixing areas (20) have clips for fixing to the body tissue.

## Revendications

1. Dispositif de prophylaxie mécanique des adhérences, en particulier pour la chirurgie abdominale endoscopique, comprenant une enveloppe gonflable (12) de type coussin, une zone d'insufflation (16) placée sur l'enveloppe (12) pour gonfler l'enveloppe (12), et au moins une zone de fixation (20) pour immobiliser l'enveloppe (12) sur les tissus du corps,
**caractérisé en ce que** l'enveloppe (12) est formée d'un ballon plat s'étendant en nappe.

2. Dispositif selon la Revendication 1,
**caractérisé en ce que** le ballon plat (12) présente les zones de fixation (20) dans des angles.

3. Dispositif selon la Revendication 1 ou 2,
**caractérisé en ce que** le ballon plat (12) est constitué d'au moins deux feuilles reliées entre elles, en particulier soudées, au moins sur les bords.

4. Dispositif selon l'une des Revendications 1 à 3,
**caractérisé en ce que** le ballon plat (12) est cloisonné par des lamelles de rigidification (14) à la manière d'un matelas pneumatique.

5. Dispositif selon l'une des Revendications 1 à 4,
**caractérisé en ce que** le contour extérieur du ballon plat (12) correspond pour l'essentiel au contour de la cavité du corps dans laquelle le dispositif de prophylaxie des adhérences (10) doit être appliqué.

6. Dispositif selon l'une des Revendications précédentes,
**caractérisé en ce que** des lamelles ou bandes de feuilles sont formées sur le ballon plat (12) pour servir de zones de fixation.

7. Dispositif selon l'une des Revendications précédentes,
**caractérisé en ce que** l'enveloppe (12) a une forme allongée et présente des lamelles de rigidification (14) disposées parallèlement à son axe longitudinal.

8. Dispositif selon l'une des Revendications précédentes,
**caractérisé en ce que** l'enveloppe (12) est constituée d'une feuille en matière synthétique indéchirable, compatible avec les tissus du corps et ne stockant que peu d'eau.

9. Dispositif selon l'une des Revendications précédentes,
**caractérisé en ce qu**'un tuyau d'insufflation (16,36) est formé sur l'enveloppe (12) en tant que zone d'insufflation (16,36).

10. Dispositif selon la Revendication 9,
**caractérisé en ce que** le tuyau d'insufflation (16,36) se raccorde à l'enveloppe (12,32) à la manière d'un entonnoir.

11. Dispositif selon l'une des Revendications précédentes,
**caractérisé en ce que** les zones de fixation (20) présentent des clips destinés à l'immobilisation sur les tissus du corps.
